# EUROPÄISCHE PATENTSCHRIFT

(11) **EP 1 408 859 B1**
(45) Veröffentlichungstag und Bekanntmachung des Hinweises auf die Patenterteilung: **06.02.2008**
(21) Anmeldenummer: 02754924.5
(22) Anmeldetag: 22.07.2002
(51) Int. Cl.: A61B 17/70

(54) **VERBINDUNGSELEMENT**
CONNECTING ELEMENT
ELEMENT DE CONNEXION

(30) Priorität: 25.07.2001 DE 10136162
(43) Veröffentlichungstag der Anmeldung: 21.04.2004
(73) Patentinhaber: BIEDERMANN MOTECH GmbH, 78054 VS-Schwenningen (DE)
(72) Erfinder: BIEDERMANN, Lutz, 78048 VS-Villingen (DE); OSTERMANN, Peter, 46397 Bocholt (DE)
(74) Vertreter: Hofer, Dorothea
(86) Internationale Anmeldenummer: PCT/EP2002/008165
(87) Internationale Veröffentlichungsnummer: WO 2003/011156

(56) Entgegenhaltungen:
- EP-A- 0 578 320
- EP-A- 0 732 081
- WO-A-00/57801
- WO-A-02/30307
- FR-A- 964 924
- US-A- 5 752 955
- US-A- 5 947 966

## Beschreibung

Die Erfindung betrifft ein Verbindungselement zum Verbinden zweier zur Knochen- bzw. Wirbelstabilisierung eingesetzter stabförmigen Elemente mit einem Verbindungsteil, einen mit dem einen stabförmigen Element verbindbaren ersten Teil und einem mit dem anderen stabförmigen Element verbindbaren zweiten Teil.

Ein derartiges Verbindungsteil ist aus der WO 91/16020 bekannt. Das Verbindungsteil weist zwei zylinderabschnittsförmig ausgebildete Kanäle auf, die zur Aufnahme zweier zueinander paralleler Stäbe dienen. Eine Verbindung zueinander geneigter oder zueinander schräg verlaufender Stäbe ist damit nicht möglich.

Ferner ist ein derartiges Verbindungsteil nur für einen vorbestimmten Abstand der Stäbe einsetzbar.

In der Druckschrift EP 0578320 A1, die den nächstliegenden Stand der Technik darstellt, wird ein Kopplungselement für die zur Wirbelstabilisierung eingesetzten Stäbe vorgeschlagen. Das Kopplungselement ist zweiteilig. Jedes Teil besitzt einen Hauptkörper und einen stabförmigen Abschnitt. Die Hauptkörper nehmen jeweils einen der zu koppelnden Stäbe auf. Die stabförmigen Abschnitte können ineinander geschoben werden.

Zu diesem Zweck ist einer der stabförmigen Abschnitte rohrförmig ausgebildet und kann mittels einer Klemmung mit Schraube zusammengezogen werden, so dass der darin eingeschobene andere stabförmige Abschnitt fixiert ist.

Die Druckschrift US 5947966 A beschreibt eine Vorrichtung zum Verbinden benachbarter Stäbe bei der Wirbelstabilisierung. Die Vorrichtung ist zweiteilig ausgebildet. Die Enden der beiden Teile können ineinander geschoben und durch eine Schraube, die nach Verschraubung in Gewindebohrungen beider Teile eingreift, fixiert werden. Das Profil der ineinander geschobenen Abschnitte ist rechteckförmig.

Aufgabe der Erfindung ist es, ein Verbindungselement der eingangs beschriebenen Art zu schaffen, welches eine Anpassung an den Abstand der zu verbindenden Stäbe erlaubt und dabei eine verbesserte Sicherung der Teile gestattet.

Diese Aufgabe wird durch das in Patentanspruch 1 gekennzeichnete Verbindungselement gelöst. Weiterbildungen der Erfindung sind in den Unteransprüchen gekennzeichnet.

Ein solches Verbindungselement hat den großen Vorteil, daß die Länge des Verbindungselements während der Operation korrigiert werden kann. Das Verbindungselement hat ferner den zusätzlichen Vorteil, daß der Chirurg in der Ausrichtung der Stäbe in Abhängigkeit von der gewünschten Ausrichtung der zu verbindenden Teile frei ist.

Weitere Merkmale und Zweckmäßigkeiten der Erfindung ergeben sich aus der Beschreibung von Ausführungsbeispielen anhand der Figuren. Von den Figuren zeigen:
- Fig. 1: eine perspektivische Darstellung eines Anwendungsfalles;
- Fig. 2: eine Schnittdarstellung durch ein erstes, nicht erfindungsgemäßes Beispiel ein Verbindungselements; und
- Fig. 3: eine Schnittdarstellung durch ein zweites, nicht erfindungsgemäßes Beispiel eines Verbindungselements;
- Fig. 4: eine Schnittdarstellung durch eine abgewandelte Ausführungsform eines nicht erfindungsgemäßen Beispieles;
- Fig. 5: eine Draufsicht auf das erfindungsgemäße Verbindungsteil des Verbindungselements ;
- Fig. 6: eine Schnittdarstellung des Verbindungsteils von Fig. 5 entlang der Linie A-A; und
- Fig. 7: eine perspektivische Darstellung der Elemente des Verbindungsteils von Fig. 5.

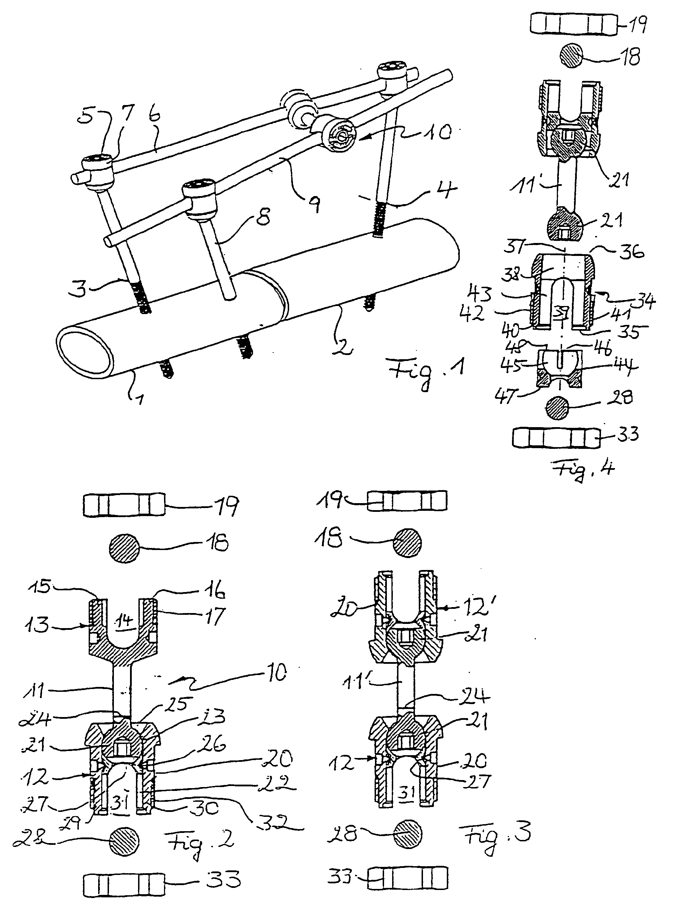

In Fig. 1 sind zwei miteinander zu verbindende und gegeneinander zu fixierende Röhrenknochenteile 1 gezeigt. In jedes der beiden Teile ist eine aus der Wirbelsäulenchirurgie als Predikelschraube bekannte Schraube 3, 4 eingeschraubt. Jede der Schrauben weist einen Kopf 5 mit einer Aufnahmeöffnung zum Aufnehmen eines Stabes 6 auf. Mit Hilfe einer Mutter 7 ist der Stab mit dem Kopf fest verbunden. Wie aus Fig. 1 ersichtlich ist, ist in dem Teil 1 eine dritte Schraube 8 eingeschraubt, die genau wie die ersten beiden Schrauben ausgebildet ist und einen zweiten Stab 9 in ihrem Kopf aufnimmt.

Der zweite Stab 9 ist über ein Verbindungselement 10 mit dem ersten Stab 6 verbunden. Dadurch wird eine zusätzliche Stabilisierung eingerichtet.

In dem obigen Ausführungsbeispiel sind die beiden zu verbindenden Teile 1 und 2 Röhrenknochenteile. In gleicher Weise erfolgt eine Anwendung von wenigstens 2 Stäben mit entsprechenden Schrauben zur Aufnahme der selben bei der Wirbelsäulenchirurgie, etwa bei einem Fixateur intern, wie er beispielsweise aus der DE 38 23 737-C bekannt ist.

Das Verbindungselement 10 weist in der in Fig. 2 gezeigten Ausführungsform einen Schaft 11 mit einem ersten Teil 12 an seinem einen Ende und einem zweiten Teil 13 an seinem anderen Ende auf.

Das zweite Teil 13 umfaßt zwei einen U-förmigen Kanal 14 begrenzende Schenkel 15, 16. Die beiden Schenkel 15, 16 weisen außen eine zylindrische Form auf und besitzen an ihren freien Enden ein Außengewinde 17. Der U-förmige Kanal 14 weist einen Durchmesser auf, der im wesentlichen gleich oder wenig größer als der Durchmesser eines aufzunehmenden Stabes 18 ist und der gerade so groß gewählt ist, daß der Stab 18 in den U-förmigen Kanal 14 einführbar und von diesem in seitlicher Richtung geführt ist. Ferner ist eine Mutter 19 vorgesehen, die mit ihrem Innengewinde so ausgestattet ist, daß sie mit dem Außengewinde 17 zusammen wirkt. Das Gewinde 17 ist so ausgebildet, daß der Abstand von dem Gewinde zum Grund des U-förmigen Kanals 14 kleiner ist, als der Durchmesser des Stabes 18, so daß der in den U-förmigen Kanal 14 eingelegte Stab 18 durch Aufschrauben der Mutter 19 fixierbar ist.

Wie aus Fig. 2 ersichtlich ist, weist das andere Ende des Stabes 18 einen einen sphärischen Abschnitt aufweisenden Kopf 21 auf. Das erste Teil 12 weist eine zylindrisch ausgebildete Fassung 20 auf, deren Durchmesser im wesentlichen gleich bzw. ein wenig größer als der Durchmesser des Kopfes 21 ist und so bemessen ist, daß der Kopf in die Bohrung einführbar und von dieser geführt ist.

In dem gezeigten Ausführungsbeispiel weist die Bohrung 22 einen Grund 23 auf, der sphärisch ausgebildet ist, wobei sein Radius gleich dem Radius des Kopfes 21. Wie aus den Figuren 2 und 3 ersichtlich ist, ist der Schaft 11 über eine Schraubverbindung von dem kugelförmigen Kopf abtrennbar ausgebildet.

Einer der beiden Teile weist eine Gewindebohrung auf, während der andere Teil einen Schraubenteil aufweist, der in die Gewindebohrung einschraubbar ist. Diese Verbindung ist schematisch durch die Verbindungsstelle 24 angedeutet. Die Gewindebohrung kann auch im Kopf 21 direkt vorgesehen sein, und der Schaft 11 hat dann an seinem dem Kopf zugewandten Ende einen entsprechend ausgebildeten Schraubansatz, der mit der Gewindebohrung zum Zusammenschrauben der beiden Teile in Eingriff bringbar ist.

In der in Fig. 2 gezeigten Ausführungsform ist zunächst der Schaft 11 von dem Kopf 21 abgeschraubt, so daß der Kopf 21 von dem freien Ende der Bohrung 22 in diese in die gezeigte Stellung einführbar ist. Es wird dann der Schaft 11 angeschraubt, so daß die in der Figur gezeigte Verbindung hergestellt ist.

Wie Fig. 2 ferner erkennen läßt, grenzt an den Grund 23 eine zweite Bohrung 25 an die trichterförmig ausgeweitet ist, so daß der Schaft 11 um einen Raumwinkel relativ zur Fassung 20 schwenkbar ist.

Nach dem eingeführten Kopf 21 wird in die Bohrung 22 ein Druckelement 26 eingesetzt, welches auf seiner dem Kopf zugewandten Seite sphärisch ausgebildet ist mit einem Radius, der dem Radius des Kopfes 21 entspricht. Auf seiner dem Kopf 21 abgewandten Seite weist das Druckelement eine zylindrische Oberfläche 27 auf, bei der der Durchmesser dieses Zylinders gleich dem Durchmesser des aufzunehmenden Stabes 28 ist, so daß der Stab 28, in die Ausnehmung 27 einlegbar ist. Wie aus Fig. 2 ersichtlich ist, ist die Ausnehmung ferner so ausgebildet, daß ein zu der Bohrung 22 koaxialer, U-förmiger Kanal 31 mit diesen begrenzenden Schenkeln 29, 30 gebildet ist. Die beiden Schenkel 29, 30 weisen wie das zweite Teil 13 angrenzend an ihre freien Enden ein Außengewinde 32 auf, welches sich soweit zu dem anderen Ende der Fassung 20 hin erstreckt, daß sein Abstand von dem Boden der zylindrischen Ausnehmung kleiner ist als der Durchmesser des Stabes 28. Ferner ist eine Mutter 33 vorgesehen, deren Innengewinde dem Außengewinde 32 entspricht.

Im Betrieb wird zunächst der Stab 18 in das monoaxial wirkende zweite Teil 13 bzw. in dessen U-förmigen Kanal 14 eingesetzt. Dann wird der Stab mit Hilfe der Mutter 19 fixiert. Anschließend wird die Fassung 20 so ausgerichtet, daß sie den zweiten Stab 28 in sich aufnimmt. Nach Einführen des Stabes 18 wird die Mutter 33 aufgeschraubt. Diese fixiert nicht nur den Stab 28, sondern übt über den Stab 28 und das Druckelement 26 auch einen solchen Druck auf den Kopf 21 auf, daß dieser in seiner Achslage stabilisiert wird.

Bei der in Fig. 3 gezeigten Ausführungsform stimmen die Fassung 20, das Druckelement 26, der Kopf 21 der Stab 28 und die Mutter 33 in allen Einzelheiten mit dem ersten Teil 12 in Fig. 2 überein. Die Ausführungsform unterscheidet sich lediglich dadurch, daß das zweite Teil nicht als monoaxiale Verbindung sondern als eine ebenfalls polyaxiale Verbindung ausgebildet ist. Dieses zweite Teil 12' ist zu dem ersten Teil 12 spiegelsymetrisch ausgebildet. Alle Teile von Fassung, Kopf, Druckelement, Stab und Mutter stimmen mit den entsprechenden Teilen des ersten Teiles 12 überein. Zur Montage ist der Schaft 11 wie in dem vorher beschriebenen Ausführungsbeispiel durch eine Schraubverbindung entlang der Linie 24 lösbar. Nach Einsetzen der Köpfe 21 werden die beiden Teile des Schaftes 11 fest miteinander verbunden.

Im Betrieb erfolgt die Verbindung wie vorher anhand des ersten Teiles 12 beschrieben, wobei die Fassung vor dem Fixieren jeweils auf den aufzunehmenden Stab hin orientiert wird und dann der Stab eingelegt und über die Mutter fixiert wird. Durch Ausüben des Druckes von der Mutter über den Stab und das Druckelement wird dann auch der Kopf endgültig fixiert.

In einer nicht gezeigten abgewandelten Ausführungsform kann die Fassung 20 bzw. das zweite Teil 13 jeweils an seinem freien Ende angrenzend noch ein Innengewinde aufweisen, in welches eine Innenschraube einschraubbar ist, um auf diese Weise in an sich bekannter Art eine Blockierung der Verschraubung zu erreichen.

Bei den oben beschriebenen Ausführungsformen ist der Kopf 21 jeweils vom freien Ende der U-förmigen Ausnehmung her eingesetzt. In einer abgewandelten Ausführungsform kann die Bohrung 22 sich durch die gesamte Fassung erstrecken. Der Kopf wird dann von dem den Schenkeln abgewandten Ende der Bohrung 22 her eingesetzt und mit einem anzubringenden Sperring oder Sprengring in der Bohrung gehalten. Entscheidend ist, daß der freie Rand, der an dem dem Schaft 11 zugewandten Ende den Kopf umgibt, kegelförmig aufgeweitet ist, um eine polyaxiale Bewegung zwischen Fassung und Kopf bzw. Schaft zu ermöglichen.

Die in Fig. 4 gezeigte Ausführungsform stimmt bezüglich Schaft 11', Kopf 21, Stab 18 bzw. 28 und Mutter 19 bzw. 33 vollständig mit der in Fig. 3 beschriebenen Ausführungsform überein. Anders ausgebildet ist die Fassung 34. Diese weist ein erstes Ende 35 und ein diesem gegenüberliegendes zweites Ende 36 auf. Es ist eine zur Symmetrieachse 37 der Fassung koaxiale Bohrung 38 vorgesehen. Ferner ist eine von dem ersten Ende 35 ausgehende U-förmige Ausnehmung 39 vorhanden, die sich quer zur Symmetrieachse 39 erstreckt. Die U-förmige Ausnehmung wird von zwei Schenkeln 40, 41 begrenzt. Angrenzend an das erste Ende 35 weisen die beiden Schenkel 40. 41 ein Außengewinde 42 auf. Der Durchmesser der U-förmigen Ausnehmung 39 ist gleich bzw. ein wenig größer als der Durchmesser des Stabes 18 bzw. 28 und gerade so groß, daß der Stab in die Ausnehmung einführbar ist und von den Seiten der U-förmigen Ausnehmung seitlich geführt ist. Das Außengewinde 42 ist wie bei den zuvor beschriebenen Ausführungformen so weit zum Grund der U-förmigen Ausnehmung ausgebildet, daß der Abstand zum U-förmigen Grund kleiner ist als der Durchmesser des aufzunehmenden Stabes. Ferner ist wie bei dem zuvor beschriebenen Ausführungsbeispiel eine Mutter 33 vorgesehen, die mit dem Außengewinde 42 zusammenwirkt.

Anders als dem zuvor beschriebenen Ausführungsbeispielen erstreckt sich der zylindrische Abschnitt 43 der koaxialen Bohrung bis zu einem vorgegebenen Abstand zum zweiten Ende 36 und ist von da ab zum zweiten Ende 36 hin mit einem Kegelwinkel konisch verjüngt. Ferner ist ein Druckelement 44 vorgesehen, dessen Außenfläche in einem den Kopf 21 seitlich umfassenden Bereich 45 gegen das zweite Ende 36 hin kegelig ausgebildet ist, wobei der Kegelwinkel dem des kegeligen Bereiches der Bohrung entspricht. Der kegelige Bereich weist eine zum zweiten Ende 36 hin gerichtete und zu diesem Ende hin offen verlaufende Schlitzung 46 auf. Durch die Anpassung der Kegelflächen zwischen Bohrung 38 und Druckelement 44 folgt bei vollständig eingeschobenem Zustand eine Selbsthemmung. Das Druckelement weist in der aus der EP 0 732 081 B bekannten Weise ein erstes Ende 47 und ein diesem gegenüberliegendes zweites Ende 48 auf. Angrenzend an das erste Ende ist ein im wesentlichen zylinderförmiger Abschnitt vorgesehen, dessen Außendurchmesser so gewählt ist, daß das Druckelement in dem zylindrischen Abschnitt 43 gleiten kann. Wie aus der Figur ersichtlich ist, weist das Druckelement in seinem zweiten Bereich eine zu dem zweiten Ende hin offene kugelsegmentförmige Ausnehmung zur Aufnahme des Schraubenkopfes auf. Im übrigen stimmen die Fassung 34 und das Druckelement 44 mit der Offenbarung in der genannten EP 0 732 081 überein, die hiermit zum Bestandteil der Beschreibung gemacht wird.

Die in Fig. 4 gezeigte Einrichtung ist spiegelsymmetrisch ausgebildet, so daß das andere Ende des Schaftes 11' mit seinem Kopf, der Fassung und dem Druckelement identisch mit dem zuvor beschriebenen ausgebildet sind.

Im Betrieb wird das Druckelement von dem ersten Ende 35 her in die Fassung 34 eingeschoben. Von dem zweiten Ende 36 her wird der Kopf 21 in den Bereich 45 eingeschoben bzw. hineingedrückt. Dann wird der Stab 28 in den verbleibenden U-förmigen Schlitz eingelegt, und über das Aufschrauben der Außenmutter 33 wird Druck auf den Stab 28 und über diesen auf das dem Kopf 21 umfassende Druckelement 44 so ausgeübt, daß Stab 28 und Kugelkopf 21 fixiert werden. Entsprechend wird auf der gegenüberliegenden Seite vorgegangen.

Auch diese Version läßt eine Schwenkung des Schaftes 11 um einen vorgegebenen Rahmenwinkel und die Symmetrieachse 37 zu, so daß eine Ausrichtung bzw. Anpassung auf die zu verbindenen Stäbe bzw. Stäbe mit Schrauben möglich wird.

Alternativ kann die Vorrichtung auch so ausgebildet sein, daß ein Kopf mit Fassung in der in Fig. 4 gezeigten Weise ausgebildet ist und der andere Kopf in der in Fig. 2 beschriebenen monoaxialen Weise geformt ist.

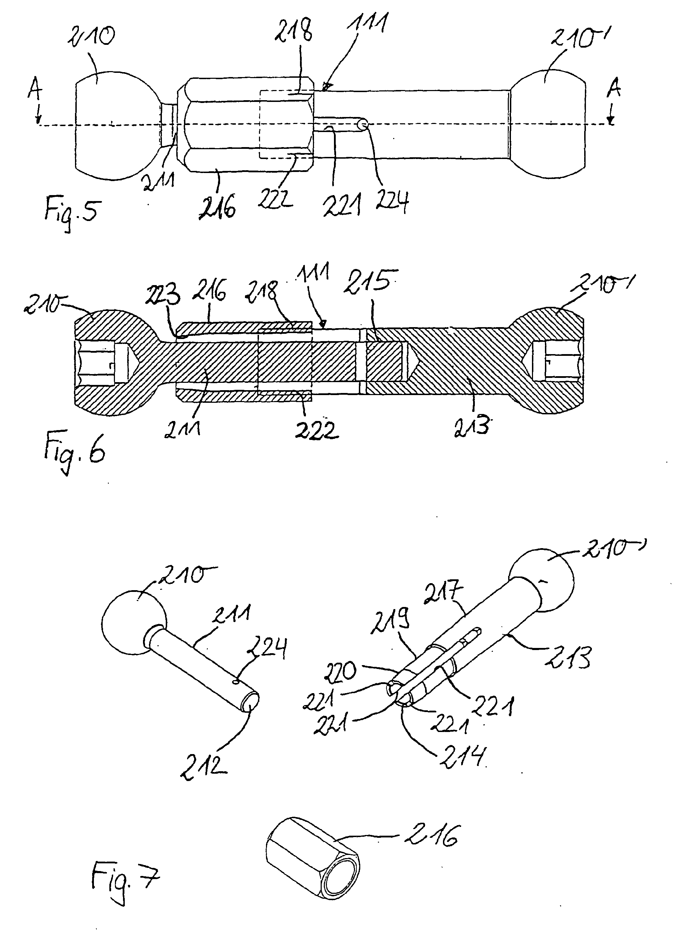

Die in den Figuren 5 bis 7 gezeigte erfinderische Ausführungsform unterscheidet sich von der in den Figuren 3 und 4 gezeigten Ausführungsform durch die Ausbildung des Schaftes 111, dessen Länge in gewissem Umfang an den Abstand der Stäbe anpassbar ist. Ansonsten stimmt diese Ausführungsform in allen Einzelheiten bezüglich der Köpfe, sowie der Teile 12, 12', der Stäbe 18, 28 und der Muttern 19, 33 mit der Ausführungsform nach Fig. 3 und bezüglich der Fassungen 34, der Stäbe 18, 28 und der Muttern 19, 33 mit der Ausführungsform nach Fig. 4 überein.

Wie insbesondere aus Fig. 7 ersichtlich ist, besteht der Schaft 111 aus drei Elementen. Ein erstes Schaftteil 211 wird aus einem mit dem einen Kopf 210 verbundenen stiftförmigen Abschnitt mit einem freien Ende 212 gebildet. Der Durchmesser des stiftförmigen Abschnittes ist so, daß der stiftförmige Abschnitt durch die Bohrung 22 der Fassung 12 hindurchführbar ist. Ein zweites Schaftteil 213 ist angrenzend an den anderen Kopf 210' im wesentlichen zylindrisch ausgebildet und weist eine sich von dem freien Ende 214 bis zu einem Abstand von dem Kopf 210' erstreckende koaxiale Bohrung 215 zur Aufnahme des ersten Schaftteiles 211 auf. Zur Fixierung der Verbindung zwischen erstem und zweiten Schaftteil ist eine später im Detail beschriebene Mutter 216 vorgesehen. Das zweite Schaftteil 213 weist angrenzend an den Kopf 210' einen ersten zylindrischen Abschnitt 217 mit einem ersten Außendurchmesser auf, der so bemessen ist, daß das Schaftteil 213 durch die Bohrung 22 der Fassung 12' hindurchführbar ist. An seinem dem Kopf 210' abgewandten Ende besitzt der erste zylindrische Abschnitt 217 ein Außengewinde 218, das in den Figuren 5 und 6 angedeutet ist. Angrenzend an den ersten zylindrischen Abschnitt 217 weist das zweite Schaftteil einen zweiten zylindrischen Abschnitt 219 auf mit einem Außendurchmesser, der kleiner ist als der des ersten zylindrischen Abschnitts. Angrenzend an den zweiten zylindrischen Abschnitt 219 weist das zweite Schaftteil einen dritten Abschnitt 220 auf, dessen Außenwand sich mit einem Kegelwinkel gegen das freie Ende 214 hin konisch verjüngt. Ferner sind in dem zweiten Schaftteil 213 wenigstens zwei, in dem gezeigten Ausführungsbeispiel vier, sich von dem freien Ende 214 bis in den ersten zylindrischen Abschnitt 217 und über den Bereich des Außengewindes 218 hinaus jedoch nicht bis zum Grund der Bohrung 215 erstreckende Längsschlitze 221 vorgesehen. Jeweils zwei Längsschlitze 221 liegen einander gegenüber. Durch die Längsschlitze sind an dem zweiten Schaftteil Zungen gebildet, die in gewissem Maße elastisch sind, so daß mit der nachfolgend beschriebenen Mutter 216 eine Klemmwirkung auf das eingeführte erste Schaftteil 211 erzielbar ist.

Die Mutter 216 ist eine Überwurf- bzw. Spannmutter. Sie ist länglich ausgebildet und hat angrenzend an ihr eines Ende ein Innengewinde 222, das mit dem Außengewinde 218 des zweiten Schaftteiles 213 zusammenwirkt. Angrenzend an ihr gegenüberliegendes Ende weist die Mutter einen Abschnitt 223 auf, dessen Innenwand sich gegen das zweite Ende der Mutter hin konisch verjüngt. Die Länge in Richtung der Längsachse der Mutter und der Kegelwinkel entsprechen dem des zweiten Schaftteils. Die Länge der Mutter ist so bemessen, daß in auf das zweite Schaftteil 213 aufgeschraubtem Zustand der konische Bereich 223 der Mutter mit dem konischen Bereich 220 des zweiten Schaftteils zusammenwirkt.

Zum Sichern gegen Verdrehen des ersten Schaftteils 211 gegenüber dem zweiten Schaftteil 213 weist das erste Schaftteil 211 in einem Abstand von seinem freien Ende 212 eine sich quer zur Schaftachse durch den stiftförmigen Abschnitt erstreckende Bohrung 224 zur Aufnahme eines entsprechenden nicht dargestellten Sicherungsstiftes auf. Der Durchmesser der Bohrung entspricht dem Durchmesser der Schlitze 221 in Querrichtung. Der einzusetzende Sicherungsstift hat eine Länge, die größer als der Durchmesser des ersten Schaftteiles 211 und maximal so groß wie der Kerndurchmesser der Mutter 216 ist.

Im Betrieb wird zuerst das erste Schaftteil 211 durch die Bohrung 22 der Fassung 12, die in Fig. 3 gezeigt ist, hindurchgeführt, bis der Kopf 210 an dem Grund 23 anliegt. Dann wird das zweite Schaftteil 213 durch die Bohrung 22 der anderen Fassung 12' hindurchgeführt, bis der Kopf 210' an dem Grund 23 anliegt. Anschließend wird die Mutter 216 ein kleines Stück auf das zweite Schaftteil 213 aufgeschraubt, so daß die konischen Abschnitte von Mutter und zweitem Schaftteil noch nicht zusammenwirken und das erste Schaftteil einführbar ist. Nun wird das erste Schaftteil 211 in die Bohrung 215 des zweiten Schaftteiles 213 soweit eingeführt, bis eine gewünschte Gesamtlänge des Schaftes hergestellt ist. Die Mutter 216 wird aber noch nicht festgezogen, so daß eine Längenkorrektur möglich ist. Zum Sichern gegen Verdrehen wird anschließend der oben beschriebene Sicherungsstift durch die Längsschlitze 221 und die Bohrung 224 hindurchgeführt. Solange die Mutter 216 noch nicht festgezogen ist, kann die Länge des Schaftes 111 auch mit eingesetztem Sicherungsstif noch verändert werden, da dieser zusammen mit dem ersten Schaftteil 211 in den Schlitzen 221 verschiebbar ist. Somit kann eine Längenkorrektur ohne Verdrehen der Köpfe 210 bzw. 210' erfolgen.

Die Verbindung der Fassungen 12, 12' mit den Stäben 18, 28 erfolgt sodann wie bei der im Zusammenhang mit der Ausführungsform nach Fig. 3 beschriebenen Weise.

Abschließend wird die Mutter 216 festgezogen. Durch das Festziehen der Mutter bis die konischen Abschnitte von Mutter und zweitem Schaftteil zusammenwirken, werden die oben beschriebenen in dem ersten Schaftteil 213 durch die Längsschlitze 221 gebildeten Zungen zusammengedrückt und klemmen dadurch das erste Schaftteil ähnlich wie bei einer Spannzange fest. Wenn sich herausstellt, daß noch eine Längenkorrektur des Schaftes 111 erforderlich ist, muß nur die Mutter 216 gelockert werden, um die Klemmwirkung der Zungen aufzuheben. In der Praxis schraubt der Chirurg erst die Schrauben an den gewünschten Positionen in den Knochen und legt dann die Stäbe ein. Das Verbindungsteil 10 ist vor dem Einbringen in der Regel bereits vormontiert, d.h. die Köpfe befinden sich in den jeweiligen Fassungen und das Schaftteil 211 ist in das zweite Schaftteil 213 eingesteckt, so daß der Schaft eine vorbestimmte Länge hat. Der Chirurg setzt dann das Verbindungsteil zwischen die zu verbindenden Stäbe und paßt die gewünschte Länge des Schaftteils wie oben beschrieben an. Dadurch kann beispielsweise auch ein Stab gegen den anderen gezogen werden.

Wenn die in den Figuren 5 bis 7 gezeigte Ausführungsform des Schaftes 111 mit der in Fig. 4 gezeigten Ausführungsform der Fassungen 34 verwendet wird, kann die Verbindung der Schaftteile erfolgen, bevor die Köpfe 210, 210' mit den Fassungen 34 verbunden werden, weil die Schaftteile mit der Kopfseite in die Fassungen eingeführt werden.

Die in den Figuren 5 bis 7 gezeigte Ausführungsform ist auch bei der in Fig. 2 gezeigten Ausführungsform anwendbar, wobei dann einer der Köpfe durch das Teil 13 ersetzt ist und das Verbindungselement dann eine monoaxiale und eine polyaxiale Verbindung hat.

Bei den oben beschriebenen Ausführungsbeispielen sind die Verbindungselemente stets zur Verbindung von Stäben erläutert. In gleicher Weise können auch zwei Schäfte von zwei Schrauben oder ein Schaft einer Schraube und ein Stab mit dem Verbindungselement verbunden und fixiert werden.

## Patentansprüche

1. Verbindungselement zum Verbinden zweier zur Knochen- bzw. Wirbelstabilisierung eingesetzter stabförmiger Elemente (6, 9, 18, 28, 3, 4, 8), mit
einem mit dem einen stabförmigen Element (28) verbindbaren ersten Teil (12; 34) und einem mit dem anderen stabförmigen Element (18) verbindbaren zweiten Teil (13, 12'; 34) und
einem Verbindungsteil mit einer Verbindungsachse zur Verbindung des ersten Teils mit dem zweiten Teil (11, 11', 111), das zwei getrennte gegeneinander verschiebbare Schaftteile (211, 213) aufweist, die so miteinander verbunden sind, dass die Länge des Verbindungsteils (11, 11', 111) in Richtung der Verbindungsachse einstellbar ist,
eine Verdrehsicherung (224, 221) zum Verhindern einer Verdrehung der beiden Schaftteile (211, 213) des Verbindungsteils gegeneinander,
**dadurch gekennzeichnet, dass** ein erstes Schaftteil (211) im wesentlichen stiftförmig ausgebildet ist, und ein zweites Schaftteil (213) nach Art einer Spannzange zur Aufnahme des ersten Schaftteils (211) ausgebildet ist, und eine Spannmutter (216) zum Fixieren der Verbindung vorgesehen ist.

2. Verbindungselement nach Anspruch 1, **dadurch gekennzeichnet, dass** die zwei Schaftteile (211, 213) über eine Klemmeinrichtung (219, 220, 216) miteinander verbunden sind.

3. Verbindungselement nach einem der Ansprüche 1 bis 2, **dadurch gekennzeichnet, dass** das zweite Schaftteil (213) im wesentlichen zylindrisch ausgebildet ist mit einer Bohrung (215) zur Aufnahme des ersten Schaftteils (211) und sich von seinem freien Ende (214) erstreckende Längsschlitze (221) aufweist, durch die Zungen gebildet werden, die unter Einwirkung der Spannmutter (216) das erste Schaftteil (211) festklemmen.

4. Verbindungselement nach einem der Ansprüche 1 bis 3, **dadurch gekennzeichnet, dass** die Verdrehsicherung einen Sicherungsstift umfasst, der sich durch wenigstens einen der Längsschlitze (221) und durch eine Bohrung in dem ersten Schaftteil (211) quer zur Schaftachse erstreckt und der bei der Längeneinstellung des Schafts in dem Längsschlitz (221) verschiebbar ist.

5. Verbindungselement nach einem der Ansprüche 1 bis 4, **dadurch gekennzeichnet, dass** das erste Teil (12, 12') polyaxial ausrichtbar und das zweite Teil (13) monoaxial mit dem Verbindungsteil (11) verbunden ist.

6. Verbindungselement nach einem der Ansprüche 1 bis 4, **dadurch gekennzeichnet, dass** beide Teile (12, 12') polyaxial ausrichtbar mit dem Verbindungsteil (11, 11' 111) verbunden sind.

7. Verbindungselement nach Anspruch 5 oder 6, **dadurch gekennzeichnet, dass** das Verbindungsteil (11, 11' 111) an seinem dem stabförmigen Element (28) zugewandten Ende einen Kopf (21, 210) und die die polyaxiale Ausrichtung ermöglichende Einrichtung (12, 12') ein Aufnahmeelement (20) mit einer Aufnahmeöffnung (31, 22) zum Aufnehmen des stabförmigen Elementes (28) einerseits und des Kopfes (21, 210) andererseits und eine das stabförmige Element (28) fixierende Mutter (33) bzw. Schraube aufweist.

8. Verbindungselement nach einem der Ansprüche 1 bis 7, **dadurch gekennzeichnet, dass** die stabförmigen Elemente als Stäbe (6, 9) ausgebildet sind.

## Claims

1. Connecting element for connecting two rod-shaped elements (6, 9, 18, 28, 3, 4, 8) used for bone or vertebra stabilisation with
a first part (12; 34), connectable to one of the rod-shaped elements (28), and a second part (13, 12' ; 34), connectable to the other rod-shaped element (18) and
a connecting part (11, 11', 111) with a connecting axis for connecting the first part with the second part, which connecting part has two separate shank parts (211, 213), displaceable in respect of one another, which are connected to one another in such a way that the length of the connecting part (11, 11', 111) is adjustable in the direction of the connecting axis,
a securing device against twisting (224, 221) to prevent twisting of the two shank parts (211, 213) of the connecting part in respect of one another,
**characterised in that** a first shank part (211) is substantially constructed as pin-shaped and a second shank part (213) is constructed in the manner of a collet chuck for receiving the first shank part (211) and a tensioning nut (216) is provided for fixing the connection.

2. Connecting element according to claim 1, **characterised in that** the two shank parts (211, 213) are connected to one another via a clamping device (219, 220, 216).

3. Connecting element according to either of claims 1 and 2, **characterised in that** the second shank part (213) is substantially constructed as cylindrical with a bore (215) for receiving the first shank part (211) and has longitudinal slots (221) extending from its open end (214), by which tongues are formed which clamp the first shank part (211) by the action of the tensioning nut (216).

4. Connecting element according to one of claims 1 to 3, **characterised in that** the security device against twisting comprises a securing pin which extends through at least one of the longitudinal slots (221) and through a bore in the first shank part (211) crosswise to the shank axis and is displaceable in the longitudinal slot (221) during length adjustment of the shank.

5. Connecting element according to one of claims 1 to 4, **characterised in that** the first part (12, 12') is connected to the connecting part (11) as polyaxially alignable and the second part (13) is monoaxially connected.

6. Connecting element according to one of claims 1 to 4, **characterised in that** both parts (12, 12') are connected to the connecting part (11, 11', 111) as polyaxially alignable.

7. Connecting element according to claim 5 or 6, **characterised in that** the connecting part (11, 11', 111) has a head (21, 210) on its end facing the rod-shaped element (28) and the device (12, 12') enabling the polyaxial alignment has a receiving element (20) with a receiving orifice (31, 22) for receiving the rod-shaped element (28) on the one hand and the head (21, 210) on the other hand and a nut (33) or screw fixing the rod-shaped element (28).

8. Connecting element according to one of claims 1 to 7, **characterised in that** the rod-shaped elements are constructed as rods (6, 9).

## Revendications

1. Élément de connexion pour relier deux éléments (6, 9, 18, 28, 3, 4, 8) en forme de barre, utilisés pour la stabilisation des os ou des vertèbres, comportant :
une première partie (12 ; 34), susceptible d'être reliée au premier élément (28) en forme de barre, et une deuxième partie (13, 12' ; 34), susceptible d'être reliée à l'autre élément en forme de barre, et
une partie de liaison (11, 11', 111), avec un axe de liaison pour assurer la liaison de la première partie à la deuxième partie, partie de liaison présentant deux parties de tiges (211, 213) séparées, déplaçables l'une par rapport à l'autre, reliées ensemble de manière que la longueur de la partie de liaison (11, 11', 111) soit réglable dans la direction de l'axe de liaison,
une sécurité de blocage en rotation (224, 221) pour empêcher une rotation des deux parties de tige (211, 213), de la partie de liaison l'une par rapport à l'autre,
**caractérisé en ce qu'**une première partie de tige (211) est réalisée sensiblement en forme de téton et une deuxième partie de tige (213) est réalisée à la façon d'une pince de serrage pour recevoir la première partie de tige (211), et un écrou de serrage (216) est prévu pour fixer la liaison.

2. Élément de connexion selon la revendication 1, **caractérisé en ce que** les deux parties de tige (211, 213) sont reliées ensemble par l'intermédiaire d'un dispositif de serrage (219, 220, 216).

3. Élément de connexion selon l'une des revendications 1 à 2, **caractérisé en ce que** la deuxième partie de tige (213) est réalisée de façon sensiblement cylindrique et est munie d'un perçage (215) pour recevoir la première partie de tige (211) et présente des fentes longitudinales (221) s'étendant depuis son extrémité libre (214), au moyen desquelles sont formées des languettes, bloquant par serrage la première partie de tige (211) sous l'effet de l'écrou de serrage (216).

4. Élément de connexion selon l'une des revendications 1 à 3, **caractérisé en ce que** la sécurité de blocage en rotation comprend un téton de sécurité, s'étendant à travers au moins l'une des fentes longitudinales (221) et à travers un perçage ménagé dans la première partie de tige (211), transversalement à l'axe de tige, et déplaçable dans la fente longitudinale (221) lors du réglage de longueur de la tige.

5. Élément de connexion selon l'une des revendications 1 à 4, **caractérisé en ce que** la première partie (12, 12') est susceptible d'être orientée de façon polyaxiale et la deuxième partie (13) est reliée de façon monoaxiale à la partie de liaison (11).

6. Élément de connexion selon l'une des revendications 1 à 4, **caractérisé en ce que** les deux parties (12, 12') sont reliées à la partie de liaison (11, 11', 111) en étant susceptibles d'être orientées de façon polyaxiale.

7. Élément de connexion selon la revendication 5 ou 6, **caractérisé en ce que** la partie de liaison (11, 11', 111) présente sur son extrémité tournée vers l'élément (28) en forme de barre, une tête (21, 210) et le dispositif (12, 12') permettant l'orientation polyaxiale présente un élément de logement (20) avec une ouverture de logement (31, 22) pour recevoir l'élément (28) en forme de barre, d'une part, et la tête (21, 210), d'autre part, et présente un écrou (33) ou une vis, fixant l'élément (28) en forme de barre.

8. Élément de connexion selon l'une des revendications 1 à 7, **caractérisé en ce que** les éléments en forme de barre, sont réalisés sous la forme de barres (6, 9).
